**Europäisches Patentamt**

**European Patent Office**

**Office européen des brevets**

(11) Veröffentlichungsnummer: **0 150 407**
**B1**

## EUROPÄISCHE PATENTSCHRIFT

(45) Veröffentlichungstag der Patentschrift:
**11.05.88**

(51) Int. Cl.⁴: **C 07 D 233/94**

(21) Anmeldenummer: **84115527.8**

(22) Anmeldetag: **15.12.84**

(54) Verfahren zur Gewinnung von hochreinem 1(2-Hydroxiethyl)-2-methyl-5-nitroimidazol.

(30) Priorität: **10.01.84 DE 3400531**

(43) Veröffentlichungstag der Anmeldung:
**07.08.85 Patentblatt 85/32**

(45) Bekanntmachung des Hinweises auf die Patenterteilung:
**11.05.88 Patentblatt 88/19**

(84) Benannte Vertragsstaaten:
**BE CH DE FR GB IT LI**

(56) Entgegenhaltungen:
**EP - A - 0 072 919**
**DE - A - 2 001 432**
**DE - A - 2 359 625**
**DE - B - 1 470 200**
**GB - A - 1 301 225**

(73) Patentinhaber: **BASF Aktiengesellschaft,
Carl-Bosch-Strasse 38, D-6700 Ludwigshafen (DE)**

(72) Erfinder: **Dockner, Toni, Dr., Grossgasse 6,
D-6701 Meckenheim (DE)**
Erfinder: **Frank, Anton, Bannwasserstrasse 72,
D-6700 Ludwigshafen (DE)**
Erfinder: **Karn, Helmut, Sternstrasse 120,
D-6700 Ludwigshafen (DE)**

## Beschreibung

Die Erfindung betrifft, wie untenstehend näher ausgeführt ist, ein Verfahren zur Gewinnung von hochreinem 1(2-Hydroxiethyl)-2-methyl-5-nitroimidazol aus einem Reaktionsgemisch, das bei der Umsetzung von 2-Methyl-4(5)nitroimidazol mit Ethylenoxid in Ameisensäure erhalten wird.

In der EP-A-72 919 ist ein Verfahren zur Gewinnung von 1,2-Dimethyl-5-nitroimidazol von hoher Reinheit aus einer wässrigen sauren Lösung des 1,2-Dimethyl-5-nitroimidazols durch Einstellen des pH-Wertes der Lösung auf 4 bis 6,5 bei 70 bis 105 °C, Abkühlen auf unter 30 °C und Abtrennen des auskristallisierten 1,2-Dimethyl-5-nitroimidazols, beschrieben. Ferner ist aus der DE-B-14 70 200 bekannt, dass man 5-Nitroimidazole mit Ethylenoxid in Gegenwart von Ameisensäure oder Essigsäure 1-(2-Hydroxialkyl)-5-nitroimidazolen umsetzen kann.

Da man bei diesem Verfahren das Ethylenoxid für die Erzielung guter Ausbeuten im Überschuss anwendet, bereitet es grosse Schwierigkeiten, die Verfahrensprodukte in reiner Form zu isolieren, denn aus dem überschüssigen Ethylenoxid werden Polyethylenglykole oder Glykolformiate gebildet, die auf das Verfahrensprodukt eine lösungsvermittelnde Wirkung ausüben.

Zur Gewinnung des Hydroxialkyl-5-nitroimidazols aus dem Reaktionsgemisch verfährt man nach den Angaben der DE-B-14 70 200 so, dass man die Ameisensäure im Vakuum abdestilliert, zum Rückstand Wasser gibt, die dabei ausgefallene Ausgangsverbindung abfiltriert, das Filtrat alkalisch macht und das dabei auskristallisierte Reaktionsprodukt abtrennt und aus Essigsäureethylester/Methanol umkristallisiert. Zur Verbesserung der Ausbeuten wird das Filtrat der alkalischen Fällung noch mit Chloroform extrahiert.

Diese Methode ist wegen der Notwendigkeit des Einsatzes von Lösungsmitteln für die Umkristallisation und die Extraktion aufwendig. Da die Verwendung des 1(2-Hydroxiethyl)-2-methyl-5-nitroimidazols als pharmazeutisches Mittel die Herstellung eines hochreinen Produktes voraussetzt, war nach einer Methode zu suchen, die es gestattet, die Verbindung aus dem Reaktionsgemisch ohne den Einsatz organischer Lösungsmittel in hochreiner Form zu gewinnen.

Es wurde nun gefunden, dass man 1(2-Hydroxiethyl)-2-methyl-5-nitroimidazol aus einem Reaktionsgemisch, das durch Umsetzung von 2-Methyl-4(5)-nitroimidazol mit Ethylenoxid in Ameisensäure erhalten wurde, durch Abdestillieren der Ameisensäure, Ausfällen des nichtumgesetzten Ausgangsstoffes nach Wasserzugabe und Ausfällen des Verfahrensproduktes durch Zugabe alkalisch wirkender Mittel zur Mutterlauge besonders vorteilhaft und in hochreiner Form gewinnen kann, wenn man in dem wässrigen Reaktionsgemisch nach Abdestillieren der Ameisensäure und vor Abtrennung des Ausgangsstoffes durch Zugabe von Mineralsäure einen pH-Wert von 0,8 bis 1,8 einstellt, das alkalisch wirkende Mittel so zur Mutterlauge gibt, dass sich ein pH-Wert von 5 bis 12 einstellt, und das abgetrennte 1(2-Hydroxiethyl)-2-methyl-5-nitroimidazol aus einer wässrigen Lösung eines alkalisch wirkenden Mittels mit einem pH-Wert von 9 bis 14 umkristallisiert.

Nach dem neuen Verfahren erhält man ein hochreines 1(2-Hydroxiethyl)-2-methyl-5-nitroimidazol. Es war überraschend, dass man eine praktisch vollständige Abtrennung der Ausgangsverbindung und der Nebenprodukte ohne eine Extraktion und Umkristallisation aus organischen Lösungsmitteln erzielen konnte und dass man das Verfahrensprodukt allein aus wässrigen Medien und durch einmaliges Umkristallisieren aus wässriger Lösung in höchster Reinheit erhält, zumal die Löslichkeiten von Ausgangs- und Endprodukt sehr ähnlich sind.

Als Ausgangsgemische kommen Reaktionsgemische in Betracht, die man bei der an sich bekannten Umsetzung von 2-Methyl-4(5)-nitroimidazol mit Ethylenoxid in Ameisensäure erhält. Bei dieser Umsetzung verfährt man z.B. so, dass man 50 bis 75 Teile 2-Methyl-4(5)-nitroimidazol in 200 bis 280 Teilen 90 bis 100%iger Ameisensäure löst und bei Temperaturen von 30 bis 50 °C mit 20 bis 60 Teilen Ethylenoxid umsetzt. Anschliessend wird die Ameisensäure weitgehend abdestilliert, zweckmässigerweise bei Temperaturen unter 90 °C und Drücken von 10 bis 100 mbar.

Nach dem erfindungsgemässen Verfahren wird nun nach der Zugabe von Wasser, wobei man vorteilhaft die 0,8 bis 1,5fache Menge an Wasser, bezogen auf den Eindampfrückstand, zugibt, der pH-Wert des wässrigen Gemisches durch Zugabe von Mineralsäuren, bei Temperaturen bis 30 °C, auf 0,8 bis 1,8, vorzugsweise 1 bis 1,5 gestellt. Als Mineralsäuren kommen z.B. Schwefelsäure, Salpetersäure, Salzsäure oder Phosphorsäure in Betracht. Die ausgefallene, nicht umgesetzte Ausgangsverbindung trennt man ab. Man kann sie für eine erneute Synthese verwenden. Die Mutterlauge wird dann durch Zugabe von alkalisch wirkenden Mitteln, wie Alkali- und Erdalkalihydroxiden oder Ammoniak, auf einen pH von 5 bis 12, vorzugsweise von 6 bis 11 gestellt, wobei das 1(2-Hydroxiethyl)-2-methyl-5-nitroimidazol ausfällt, welches man abtrennt. Man nimmt diese Ausfällung und Abtrennung bei Temperaturen von maximal 30 °C vor, wobei man zweckmässigerweise vor der Abtrennung, z.B. durch ein Eisbad, abkühlt.

Nach einer besonders vorteilhaften Ausführungsform des erfindungsgemässen Verfahrens führt man die Ausfällung des Verfahrensproduktes und seine Abtrennung in zwei Stufen in der Weise durch, dass man den pH-Wert der Mutterlauge vor der ersten Fällung auf 5 bis 7, vorzugsweise 6 bis 7, und vor der zweiten Fällung auf 9 bis 12, vorzugsweise 9 bis 11, stellt und die vorstehend bezeichnete Maximaltemperatur einhält.

Das ausgefallene und abgetrennte Verfahrensprodukt wird aus einer wässrigen Lösung eines alkalisch wirkenden Mittels der oben genannten Art umkristallisiert, wobei der pH-Wert der wässrigen Lösung 9 bis 14, vorzugsweise 10 bis 13 betragen soll. Man kristallisiert zweckmässigerweise aus Ammoniakwasser um. Die wässrig-alkalische Mutterlauge aus dieser Umkristallisation gibt man vorteilhaft nach dem Abdestillieren der Ameisensäure zum Reaktionsgemisch.

Nach dem neuen Verfahren erhält man 1(2-Hydroxiethyl)-2-methyl-5-nitroimidazol in hoher Ausbeute und in einer Reinheit von 99,8%.

Beispiel 1

254 Teile 2-Methyl-4(5)-nitroimidazol werden in 736 Teilen 98%iger Ameisensäure gelöst. In diese Lösung werden bei 30 bis 40 °C innerhalb von 20 bis 30 Minuten unter gutem Rühren 176 Teile Ethylenoxid eingeleitet. Man rührt 2 1/2 Stunden bei 30 bis 40 °C nach, wobei am Anfang gelegentlich schwach gekühlt werden muss, und destilliert dann im Rotationsverdampfer bei 30 mbar Druck und einer Wasserbadtemperatur, die im Verlauf der Destillation auf 85 °C gesteigert wird, 550 Teile Ameisensäure ab.

Der Eindampfrückstand wird mit 600 Teilen Wasser versetzt. Die saure Lösung (pH = 3,1) wird bei 20 °C durch Zugabe von 48 Teilen konz. Schwefelsäure auf den pH 1,25 gestellt. Das Gemisch wird 1 Stunde bei 20 bis 30 °C gerührt. Dann wird der ausgeschiedene Feststoff abgesaugt und mit 50 Teilen Wasser gewaschen. Man erhält 90 Teile nicht umgesetztes 2-Methyl-4(5)-nitroimidazol. Filtrat und Waschwasser werden bei 20 °C durch Zusatz von Ammoniakwasser auf pH 6 gestellt. Man kühlt 1 Stunde im Eisbad und saugt das ausgeschiedene Endprodukt ab. Das Filtrat wird durch Zusatz von Natronlauge auf pH 10 gestellt und 1 Stunde im Eisbad gekühlt. Dann wird das ausgefallene Endprodukt (zweite Fraktion) abgesaugt.

Die beiden Fraktionen des Endprodukts werden vereint und aus 400 Teilen Wasser, das durch Zusatz von Ammoniakwasser auf pH 10 eingestellt wurde, umkristallisiert. Man erhält 180 Teile 1(2-Hydroxiethyl)- 2-methyl-5- nitroimidazol vom Schmelzpunkt 158 bis 160,2 °C und einer Reinheit von 99,9% (nach HPLC) entsprechend einer Ausbeute von 81,4%, bezogen auf umgesetztes 2-Methyl-4(5)-nitroimidazol.

Beispiel 2

90 Teile des nach Beispiel 1 zurückgewonnenen, nichtumgesetzten 2-Methyl-4(5)-nitroimidazols und 164 Teile frisches 2-Methyl-4(5)-nitroimidazol werden in 736 Teilen Ameisensäure, wie in Beispiel 1 beschrieben, mit Ethylenoxid umgesetzt. Nach Abdestillieren der Ameisensäure gemäss Beispiel 1 wird der Eindampfrückstand mit dem ammoniakalischen Filtrat versetzt, das bei der Umkristallisation der beiden Fraktionen des Endprodukts nach Beispiel 1 erhalten wurde. Das Gemisch wird nun wie in Beispiel 1 beschrieben mit Schwefelsäure auf den pH 1,25 gestellt. Dann wird weiter aufgearbeitet wie in Beispiel 1 beschrieben. Man erhält 99 Teile nicht umgesetztes 2-Methyl-4(5)-nitroimidazol und 180 Teile 1-(2-Hydroxiethyl)- 2-methyl-5- nitroimidazol mit der in Beispiel 1 angegebenen Reinheit.

**Patentansprüche**

1. Verfahren zur Gewinnung von hochreinem 1(2-Hydroxiethyl)-2-methyl-5-nitroimidazol aus einem Reaktionsgemisch, das durch Umsetzung von 2-Methyl-4(5)-nitroimidazol mit Ethylenoxid in Ameisensäure erhalten wurde, bei dem man Ameisensäure abdestilliert, nicht umgesetzten Ausgangsstoff nach Wasserzugabe ausfällt und abtrennt und das Verfahrensprodukt durch Zugabe alkalisch wirkender Mittel zur Mutterlauge ausfällt, dadurch gekennzeichnet, dass man in dem wässrigen Reaktionsgemisch nach Abdestillieren der Ameisensäure

a) durch Zugabe von Mineralsäure bei Temperaturen bis 30 °C einen pH-Wert von 0,8 bis 1,8 einstellt und den ausgefallenen Ausgangsstoff abtrennt,

b) das alkalisch wirkende Mittel so zur Mutterlauge gibt, dass sich ein pH-Wert von 5 bis 12 einstellt und eine Temperatur von 30 °C nicht überschritten wird,

c) das ausgefallene 1(2-Hydroxiethyl)-2-methyl-5-nitroimidazol abtrennt und aus einer wässrigen Lösung eines alkalisch wirkenden Mittels mit einem pH-Wert von 9 bis 14 umkristallisiert.

2. Verfahren nach Anspruch 1, dadurch gekennzeichnet, dass man das Ausfällen und Abtrennen des 1(2-Hydroxiethyl)-2-methyl-5-nitroimidazol nach Zugabe des alkalisch wirkenden Mittels in zwei Stufen durchführt, wobei man den pH-Wert vor der ersten Fällung auf 5 bis 7 und vor der zweiten Fällung auf 9 bis 12 stellt.

3. Verfahren nach Anspruch 1, dadurch gekennzeichnet, dass man die wässrig-alkalische Mutterlauge der Umkristallisation nach Abdestillieren der Ameisensäure zum Reaktionsgemisch gibt.

**Revendications**

1. Procédé pour la préparation de 1-(2-hydroxyéthyl)-2-méthyl-5-nitro-imidazole de grande pureté à partir d'un mélange réactionnel qui a été obtenu par réaction de 2-méthyl-4(5)-nitro-imidazole avec l'oxyde d'éthylène dans l'acide formique, procédé dans lequel on chasse par distillation l'acide formique, on précipite et on sépare la substance de départ n'ayant pas réagi après addition d'eau, et on précipite le produit voulu par addition d'agents à action alcaline à la liqueur mère, caractérisé en ce que dans le mélange réactionnel aqueux, après avoir chassé par distillation l'acide formique,

a) on règle un pH de 0,8 à 1,8 par addition d'acide minéral à une température allant jusqu'à 30 °C et on sépare la substance de départ précipitée,

b) on ajoute l'agent à action alcaline à la liqueur mère de telle manière qu'un pH de 5 à 12 soit réglé et qu'une température de 30 °C ne soit pas dépassée,

c) on sépare le 1-(2-hydroxyéthyl)-2-méthyl-5-nitroimidazole précipité et on le recristallise à partir d'une solution aqueuse d'un agent à action alcaline, ayant un pH de 9 à 14.

2. Procédé selon la revendication 2, caractérisé en ce qu'on effectue en deux temps la précipitation et la séparation du 1-(2-hydroxyéthyl)-2-méthyl-5-nitroimidazole après l'addition de l'agent à action alcaline, le pH étant réglé à 5–7 avant la première précipitation et à 9–12 avant la seconde précipitation.

3. Procédé selon la revendication 1, caractérisé en ce qu'on ajoute au mélange réactionnel la liqueur mère hydro-alcaline de la recristallisation après avoir chassé l'acide formique par distillation.

**Claims**

1. A process for preparing 1-(2-hydroxyethyl)-2-methyl-5-nitroimidazole of high purity from a reaction mixture obtained by reacting 2-methyl-4(5)-nitroimidazole with ethylene oxide in formic acid, wherein formic acid is distilled off, unconverted starting material is precipitated after addition of water and separated off, and the product is precipitated by adding an alkaline agent to the mother liquor, which comprises bringing the aqueous reaction mixture, after the formic acid has been distilled off,

a) to a pH from 0.8 to 1.8 at up to 30 °C by adding a mineral acid and separating off the precipitated starting material,

b) adding the alkaline agent to the mother liquor in such a way that a pH from 5 to 12 becomes established and 30 °C is not exceeded,

c) separating off the precipitated 1-(2-hydroxyethyl)-2-methyl-5-nitroimidazole and recrystallizing it from an aqueous solution of an alkaline agent having a pH from 9 to 14.

2. A process as claimed in claim 1, wherein the precipitation and removal of the 1-(2-hydroxyethyl)-2-methyl-5-nitroimidazole after addition of the alkaline agent is carried out in two stages, the pH being adjusted to 5–7 before the first precipitation and to 9–12 before the second precipitation.

3. A process as claimed in claim 1, wherein the aqueous alkaline mother liquor of the recrystallization is added to the reaction mixture after the formic acid has been distilled off.